# EUROPEAN PATENT APPLICATION

(11) **EP 2 424 196 A1**
(43) Date of publication of application: **29.02.2012**
(21) Application number: 11006000.1
(22) Date of filing: 21.07.2011
(51) Int. Cl.: H04M 1/05, H04M 1/725, G08B 21/02, G08B 25/00, G08B 25/10, H04B 1/38, A42B 3/04

(54) **Personal emergency system for a mobile communication device**

(30) Priority: 23.08.2010 US 860966
(71) Applicant: Sony Ericsson Mobile Communications AB, 221 88 Lund (SE)
(72) Inventor: Persson, Magnus, 24747 Flyinge (SE); Nyström, Martin, 28294 Hörja (SE)
(74) Representative: Banzer, Hans-Jörg

(57) **Abstract**

The occurrence of an accident is determined with the aid of a motion sensor disposed in an accessory (e.g. headset) (200) of a mobile communication device (10). The accessory (200) includes a body sensor (202) to detect that the accessory (200) is being worn, and a body movement sensor (204) to detect body movements. An accident is inferred from a violent body movement while the accessory (200) is being worn. In response to the violent body movement, an emergency message is generated and sent to a predetermined destination address.

## Description

The present invention relates generally to headsets for mobile terminals and, more particularly to headsets with integrated motion sensors for detecting violent head movements that may result in injury.

Emergency notification systems incorporating wireless transceivers are well known in the art. For example, vehicle-based emergency systems, such as On-star, may detect accidents based on sensors mounted in the vehicle and immediately send an emergency message to an emergency response center. The immediate notification decreases the response time of emergency personnel and increases the chances of an injured person surviving a critical injury.

While vehicle-based emergency systems have no doubt saved many lives, such systems have a number of limitations. First, vehicle-based emergency systems are limited to detection of car accidents. However, many accidents occur that do not involve a car collision. Second, vehicle-based emergency systems are relatively expensive and add significantly to the cost of a vehicle. Consequently, most cars in use today are not equipped with emergency systems.

Therefore, there is a continuing need for a personal emergency system that is vehicle-independent and can detect car accidents and other types of accidents.

### SUMMARY

According to the invention, a method implemented by a mobile communication device of providing an emergency notification as defined in claim 1 and an emergency system as defined in claim 8 are provided. The dependent claims define preferred embodiments of the invention. The present invention facilitates the detection of an accident with the aid of a motion sensor disposed at an accessory for a mobile communication device that is worn on the user's body, such as a headset, pedometer, body monitor, etc. The accessory includes one or more accessory sensors to detect when the accessory is being worn and to detect body movements. An accident is inferred from a violent body movement while the accessory is being worn. In response to the violent body movement, an emergency message is automatically generated and sent to a predetermined destination address even when the user is incapacitated or otherwise unable to report the accident. In one embodiment, the accessory comprises a headset including a head sensor for detecting when the headset is being worn and a head movement sensor for detecting violent head movements.

Exemplary embodiments of the present invention include methods implemented by a mobile communication device of providing an emergency notification. In one exemplary embodiment, the method comprises detecting that an accessory for the mobile communication device is being worn by a user; detecting a violent body movement while the accessory is being worn by the user; and transmitting an emergency message to a predetermined destination address responsive to the detection of a violent body movement.

Some exemplary methods may further comprise generating an audible alarm responsive to the detection of the violent body movement to get the attention of persons in the vicinity of the user.

In some exemplary methods, the emergency message includes at least one of location information, time information, and user information.

In some exemplary methods, transmitting an emergency message to a predetermined destination address comprises transmitting a short text message to said predetermined destination address.

In some exemplary methods, transmitting an emergency message to a predetermined destination address comprises transmitting a synthesized voice message to said predetermined destination address.

In some exemplary methods, transmitting an emergency message to a predetermined destination address comprises transmitting an emergency message to at least one of an emergency response center and an emergency contact designated by the user.

In some exemplary methods the accessory comprises one of a headset, a heart rate monitor, and pedometer.

Other embodiments of the present invention comprise an emergency system for a mobile communication device. One exemplary emergency system comprises an accessory that is worn on a user's body and includes one or more accessory sensors; a transmitter to transmit an emergency message; a detection circuit configured to receive signals from the accessory sensors and to detect violent body movements while the accessory being worn based on said signals from said accessory sensors; and a signaling circuit configured to generate said emergency message and to transmit said emergency message via said transmitter to a predetermined destination address responsive to the detection, by said detection circuit, of a violent body movement while said accessory is being worn.

Some exemplary embodiments of the emergency system further comprise a loudspeaker, wherein the signaling circuit is further configured to generate an audible alarm for output via said loudspeaker responsive to the detection of the violent body movement.

In some embodiments of the emergency system, the emergency message includes at least one of location information, time information, and user information.

In some embodiments of the emergency system, the signaling circuit controls said transmitter to transmit a short text message to said predetermined destination address.

In some embodiments of the emergency system, the signaling circuit controls said transmitter to transmit a synthesized voice message to said predetermined destination address.

In some embodiments of the emergency system, the signaling circuit controls said transmitter to transmit an emergency message to at least one of an emergency response center and an emergency contact designated by the user.

In some embodiments of the emergency system, the accessory comprises one of a headset, a heart rate monitor, and pedometer.

### BRIEF DESCRIPTION OF THE DRAWINGS

Figure 1 is a perspective view of a wireless communication device communicating with a headset according to one embodiment of the present invention.

Figure 2 is a block diagram illustrating the main functional components of an emergency system for a mobile communication device according to a first embodiment.

Figure 3 is a block diagram illustrating the main functional components of an emergency system for a mobile communication device according to a second embodiment.

Figure 4 illustrates an exemplary method based on a combination of mobile terminal movement and head movement.

### DETAILED DESCRIPTION

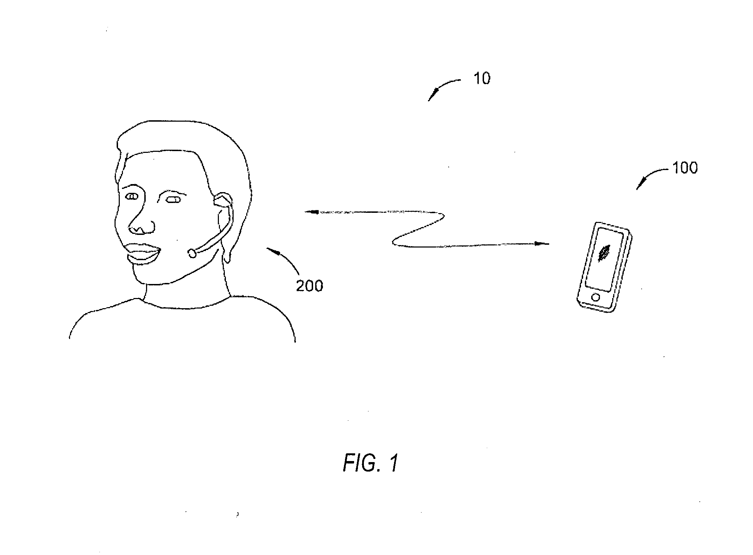

FIG. 1 illustrates a first exemplary embodiment of the mobile communication device 10 of the present invention which may be used for hands-free communication. The mobile communication device 10 comprises two main parts: a mobile terminal 100 and an accessory (e.g., headset, watch, heart rate monitor, pedometer, etc.) that 200 is worn on the user's body. The exemplary embodiment illustrates the accessory 200 as a headset, which is hereinafter referred to as headset 200. Those skilled in the art will appreciate that the invention is equally applicable to other accessories that are worn on the user's body. The mobile terminal 100 and headset 200 are preferably configured as separate units. In the exemplary embodiment shown in Fig. 1, the mobile terminal 100 comprises a cellular phone. In other embodiments, the mobile terminal 100 may comprise a smart mobile terminal, satellite phone, Personal Digital Assistant (PDA), laptop computer, notebook computer, or other device with wireless communications capabilities.

The mobile terminal and headset 200 may be connected by wire or by a wireless interface. In the exemplary embodiment, the mobile terminal 100 and headset 200 communicate with each over a wireless communication link using, for example, BLUETOOTH technology or other short-range wireless communication technology. Initially, the mobile terminal 100 and headset 200 execute a procedure to pair with each other and establish a short-range communication link. That procedure is well-known to those of ordinary skill in the art and not germane to the present invention. Therefore, it is not discussed in detail herein. When the headset 200 is paired with the mobile terminal 100, the headset 200 may exchange audio and/or control signals with the mobile terminal 100 via the wireless link.

According to the present invention, violent head movements (or violent body movements for other types of accessories) indicative of an accident may be detected with the aid of one or more sensors mounted on the headset 200. In response, the mobile communication device 10 may transmit an emergency message and/or generate an audible alarm signal in response to the accident. For example, the mobile terminal 100 may call an emergency response center and transmit a predetermined emergency message to notify emergency response personnel that an accident has occurred. The transmitted message may include location information to indicate the location of the accident. Thus, emergency response personnel can be notified even when the user is incapacitated and unable to place an emergency call. In addition, the mobile communication device 10 may be configured to generate an audible alarm to alert persons nearby who may be able to provide first aid or other emergency assistance.

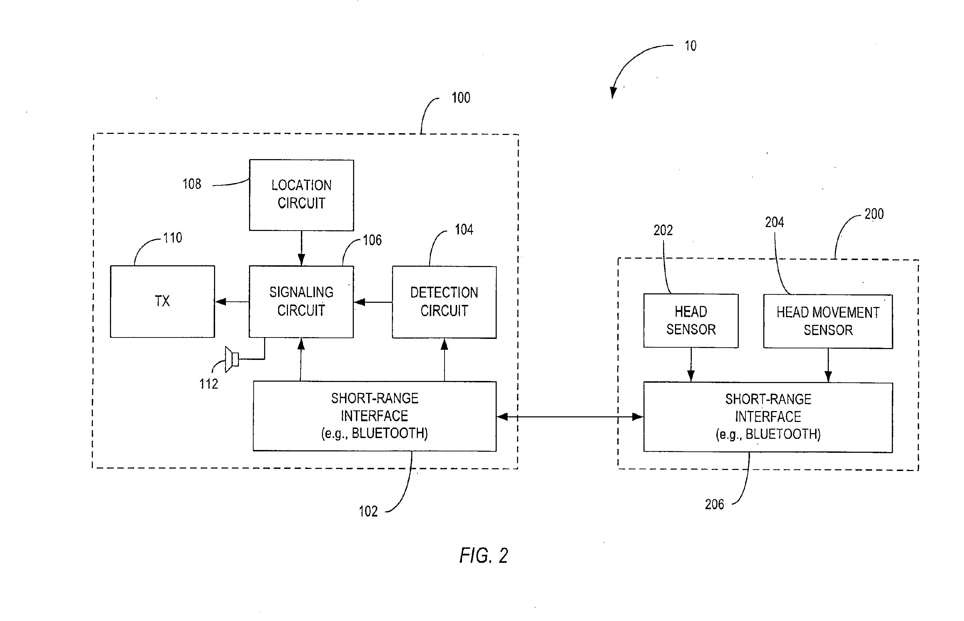

Figure 2 illustrates the main functional components of a mobile terminal 100 and a headset 200 for implementing an emergency notification function according to one exemplary embodiment. Components of the mobile terminal 100 and headset 200 not essential to the understanding of the present invention are omitted for the sake of clarity.

The headset 200 includes one or more sensors to detect when the headset is being worn by the user; and to detect violent head movements indicative of an accident. In the exemplary embodiment shown in Figure 2, the headset 200 includes a head sensor 202 to detect when the headset is being worn by the user and a separate head movement or body movement sensor 204 to detect head or body movements. The head sensor 202 may comprise a biometric sensor to detect a physiologic condition of the user, such as an ear thermometer or pulse detector, a proximity detector, a mechanical switch that closes when the headset is placed on the user's head, an orientation sensor, or accelerometer. In some embodiments, inputs from multiple sensors may be used to detect when the headset 200 is being worn.

The head movement sensor 204 preferably comprises one or more accelerometers. Either single-axis or multiple-axis accelerometers may be used. Various MEMS (micro electrical mechanical systems) accelerometers are currently available for mobile communication devices and are well-known to those skilled in the art. Gyroscopes and magnetometers may also be used to detect head and body movements. While the head sensor 202 and head movement sensor 204 are shown as separate sensors in Figure 2, those skilled in the art will appreciate that a single sensor can be used as both a head sensor and head movement sensor. Signals generated by the head sensor 202 and head movement sensor 204 are transmitted from the headset 200 to the mobile terminal 100 via a short-range wireless interface 206, such as a BLUETOOTH interface.

The mobile terminal 100 includes a short-range wireless interface 102, a detection circuit 104, signaling circuit 106, location circuit 108, and a transmitter 110. The short-range wireless interface 102 enables communication between the mobile terminal 100 and head set 200. As previously noted, the short-range wireless interface 102 may comprise a BLUETOOTH interface or other short-range radio interface. In the exemplary embodiment, the short-range wireless interface 102 receives signals from the head sensor 202 and head movement sensor 204 and applies the signals to the detection circuit 104 for analysis.

The detection circuit 104 monitors the head sensor 202 and head movement sensor 204 and determines the occurrence of an accident based on the signals from the head sensor 202 and head movement sensor 204. For example, the detection circuit 104 may enable the head movement sensor 204 when it is detected that the headset 200 is being worn. When the headset is being worn, the detection circuit 104 compares the signals from the head movement sensor 204 with a predetermined threshold to determine whether an accident has occurred. When an accident occurs that requires an emergency response, the detection circuit 104 generates an emergency signal that is applied to the signaling circuit 106.

The main purpose of the signaling circuit 106 is to generate an emergency message responsive to the detection of an accident. In response to the emergency signal from the detection circuit 104, the signaling circuit 106 generates an emergency message and activates the transmitter 110 to transmit the emergency message to a predetermined destination address. The emergency message may comprise a text message, such as a short-text message, an instant message, or an email message. Alternatively, the emergency message may comprise a synthesized voice message. The emergency message may include time information indicating the time of the accident; location information provided by the location circuit 108 indicating the location of the accident; and user information indicating the identity of the user and/or the physiologic condition of the user. For example, the user information could include the user's body temperature, heart rate, blood pressure, etc. to aid emergency personnel responding to the emergency. The signaling circuit 106 may in some embodiments include a loudspeaker 112 to generate an audible alarm for getting the attention of nearby persons who may be able to provide assistance.

The location circuit 108 determines the location of the mobile terminal 100, which may be included in the emergency message. The location circuit 108 may comprise, for example, a Global Positioning System (GPS) receiver or other Global Navigation Satellite System (GNSS) that determines the location of the mobile terminal 100 based on signals received from earth-orbiting satellites. If the mobile terminal 100 does not include a GNSS, network-based location methods can be used to determine the location of the mobile terminal 100. For example, the mobile terminal 100 could determine its location based on positioning signals transmitted from three or more base stations. Also, if the mobile terminal 100 is equipped with a BLUETOOTH interface, the mobile terminal 100 could attempt to acquire its location from a nearby device that is equipped with a GPS receiver.

When an accident is detected, the signaling circuit 106 may request the location circuit 108, network, or nearby device to provide the current location for transmission with the emergency message. Alternatively, the network could determine the location of the mobile station from the transmission of the emergency message or other positioning signal by the mobile terminal 100.

The transmitter 110 enables the mobile terminal 100 to communicate with remote devices over wireless communication networks, such as a cellular network or wireless local area network (WLAN). In some embodiments, the transmitter 110 may be part of a cellular transceiver implementing any one of a variety of communication standards including, but not limited to, the standards known as the Global System for Mobile Communications (GSM), General Packet Radio Service (GPRS), Wideband CDMA (W-CDMA), and Long-Term Evolution (LTE). The transmitter 110 may also be part of a wireless local area network (WLAN) transceiver, such as a WiFi transceiver. Other embodiments may include multiple transmitters 110 implementing different communication protocols.

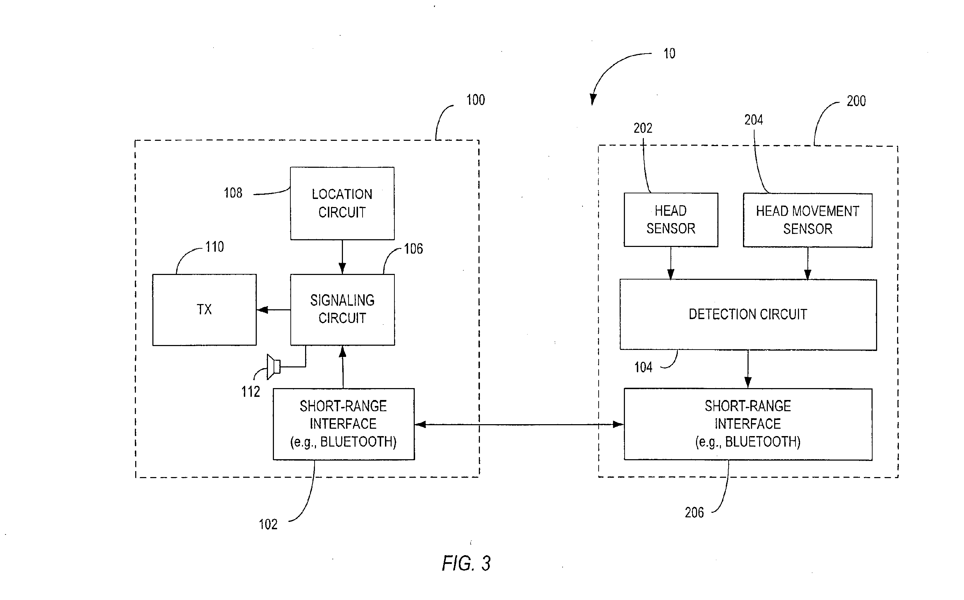

The detection circuit 104 and signaling circuit 106 may be implemented by one or more general purpose or special purpose processors. In some embodiments, a single processor may be configured by software to perform the functions of both the detection circuit 104 and signaling circuit 106. In other embodiments, the detection circuit 104 and signaling circuit 106 may be implemented in two or more processors. While the detection circuit 104 is shown as part of the mobile terminal 100 in Figure 2, those skilled in the art will appreciate that the detection circuit 104 could also be located in the headset 200 as shown in Figure 3.

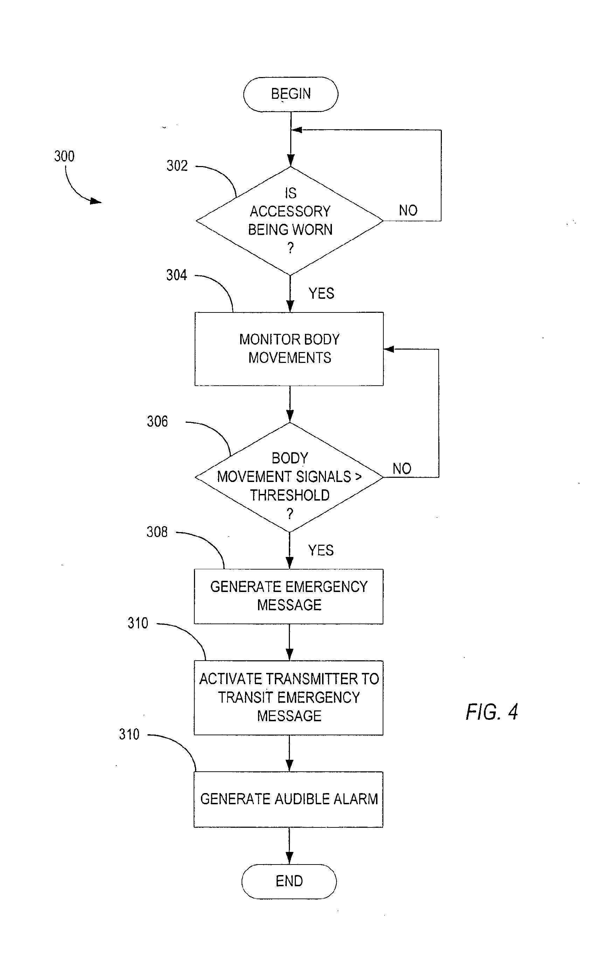

Figure 4 illustrates an exemplary method 300 of providing an emergency notification with the aid of a sensor in the headset 200 or other accessory for a mobile communication device 10. The detection circuit 104 monitors signals from the head or body sensor 202 to detect when the headset or accessory 200 is being worn (block 302). If the headset or other accessory 200 is being worn, the detection circuit 104 monitors head or body movement signals from the head or body movement sensor 204 (block 304). Monitoring the head movement or body movement sensor 204 only when the headset or other accessory 200 is worn prevents false alarms, such as when the headset or accessory 200 is dropped. The detection circuit 104 compares the head or body movement signals received when the headset or accessory 200 is being worn to a threshold (block 306). The threshold is set to a level that indicates a violent head or body movement with a potential for injury. If the head or body movement signals exceed the threshold, the detection circuit 104 generates a emergency signal indicating that an accident has occurred. In response to the emergency signal, the signaling circuit 106 generates an emergency message (block 308) and activates the transmitter to transmit the emergency message to a predetermined destination address (block 310). For example, the signaling circuit may transmit the emergency message to an emergency response center (e.g., 911 center), and/or to a predetermined emergency contact (e.g., spouse, parent, etc.) specified by the user in a user profile. Also, the signaling circuit 106 may be configured to transmit the emergency message to nearby devices via the short-range wireless interface 102. Additionally, the emergency system may activate a loudspeaker 112 to generate an audible alarm to attract the attention of nearby persons who may be able to provide first aid or other emergency assistance (block 312).

Due to the pervasiveness of mobile communication devices 10, it may be possible for emergency response centers to determine the occurrence of accidents involving large groups of people by correlating the time and/or location information in emergency message transmitted from different mobile communication devices. For example, a bus accident or building collapse may result in near simultaneous emergency messages to an emergency response center. Based on the time and/or location information in the emergency messages, the emergency response center may quickly determine that a major accident has occurred and coordinate the response of emergency personnel.

The present invention may, of course, be carried out in other specific ways than those herein set forth without departing from the scope and essential characteristics of the invention. The present embodiments are, therefore, to be considered in all respects as illustrative and not restrictive, and all changes coming within the meaning and equivalency range of the appended claims are intended to be embraced therein.

## Claims

1. A method implemented by a mobile communication device (10) of providing an emergency notification, said method comprising:
detecting that an accessory (200) for the mobile communication device (10) is
being worn on a user's body;
detecting a violent body movement while the accessory is being worn by the
user; and
transmitting an emergency message to a predetermined destination address
responsive to a detection of a violent body movement.

2. The method of claim 1 further comprising generating an audible alarm responsive to the detection of the violent body movement to get the attention of persons in the vicinity of the user.

3. The method of claim 1 or claim 2 wherein the emergency message includes at least one of location information, time information, and user information.

4. The method of any one of claims 1-3 wherein transmitting an emergency message to a predetermined destination address comprises transmitting a short text message to said predetermined destination address.

5. The method of any one of claims 1-4 wherein transmitting an emergency message to a predetermined destination address comprises transmitting a synthesized voice message to said predetermined destination address.

6. The method of any one of claims 1-5 wherein transmitting an emergency message to a predetermined destination address comprises transmitting the emergency message to at least one of an emergency response center and an emergency contact designated by the user.

7. The method of any one of claims 1-6 wherein the accessory (200) comprises one of a headset, heart rate monitor, and pedometer.

8. An emergency system for a mobile communication device (10) comprising:
an accessory (200) adapted to be worn on a user's body and including one or
more accessory sensors (202, 204);
a transmitter (110) configured to transmit an emergency message;
a detection circuit (104) configured to receive signals from said accessory
sensors (202, 204) and to detect violent body movements while the accessory (200) is being worn based on said signals from said accessory sensors (202, 204); and
a signaling circuit (106) configured to generate said emergency message and to
transmit said emergency message via said transmitter (110) to a predetermined destination address responsive to a detection, by said detection circuit (104), of a violent body movement while said accessory (200) is being worn.

9. The emergency system of claim 8 further comprising a loudspeaker (112) and wherein the signaling circuit (106) is further configured to generate an audible alarm for output via said loudspeaker (112) responsive to the detection of the violent body movement.

10. The emergency system of claim 8 or claim 9 wherein the emergency message includes at least one of location information, time information, and user information.

11. The emergency system of any one of claims 8-10 wherein the signaling circuit (106) controls said transmitter (110) to transmit a short text message to said predetermined destination address.

12. The emergency system of any one of claims 8-11 wherein the signaling circuit (106) controls said transmitter (110) to transmit a synthesized voice message to said predetermined destination address.

13. The emergency system of any one of claims 8-12 wherein the signaling circuit (106) controls said transmitter (110) to transmit said emergency message to at least one of an emergency response center and an emergency contact designated by the user.

14. The emergency system of any one of claims 8-13 wherein the accessory (200) comprises one of a headset, heart rate monitor, and pedometer.
